# FASCICULE DE BREVET EUROPEEN

(11) **EP 0 642 808 B1**
(45) Date de publication et mention de la délivrance du brevet: **29.12.1999**
(21) Numéro de dépôt: 94113821.6
(22) Date de dépôt: 03.09.1994
(51) Int. Cl.: A61N 1/30

(54) **Dispositif en trois modules pour l'administration transdermique de médicaments par électrophorèse ou iontophorèse**
Vorrichtung mit drei Modulen zur iontophoretischen oder elektrophoretischen transdermalen Abgabe von Medikamenten
Device with three modules for iontophoretics or electrophoretic transdermal drug delivery

(30) Priorité: 10.09.1993 FR 9310897
(43) Date de publication de la demande: 15.03.1995
(73) Titulaire: ASULAB S.A., CH-2501 Bienne (CH)
(72) Inventeur: Frenkel, Erik Jan, CH-2000 Neuchâtel (CH); Born, Jean-Jacques, CH-1110 Morges (CH); Schafroth, Konrad, CH-3011 Bern (CH)
(74) Mandataire: Thérond, Gérard Raymond

(56) Documents cités:
- EP-A- 0 254 965
- GB-A- 2 239 803
- US-A- 4 942 883
- US-A- 5 135 479

## Description

La présente invention concerne un dispositif de traitement ambulatoire, réalisé sous forme de trois modules séparables, pour l'administration transdermique de médicaments par électrophorèse ou iontophorèse.

Outre les méthodes classiques d'administration par voie orale ou parentérale, la méthode consistant à administrer un médicament en lui faisant franchir la barrière cutanée est connue depuis longtemps. Les médicaments susceptibles d'être administrés de cette façon peuvent être classés en deux groupes. Le premier groupe est représenté par les médicaments qui franchissent naturellement la barrière cutanée par une simple application sur la peau et qui passent directement dans la circulation sanguine. Pour une telle application, le dispositif le plus simple se présente sous forme d'un support contenant le médicament à administrer, tel qu'un gel ou un tampon imprégné, et ledit support étant maintenu en place au moyen d'une bande adhésive pendant toute la durée d'un traitement pouvant varier de vingt-quatre heures à une semaine. De tels dispositifs, et tous les perfectionnements auxquels ils ont donné lieu, sont généralement désignés sous le nom de "patch passif". Un petit nombre de médicaments se prête déjà à ce mode d'administration qui présente des avantages certains, en particulier au niveau d'une diminution de la toxicité systémique. De tels dispositifs correspondent par exemple à celui qui est décrit dans le brevet européen EP 0 336 543, pour l'administration, entre autres, de la clonidine (hypertension), ou de l'oestradiol (syndrome de la ménopause). Malgré les recherches effectuées pour faciliter ou contrôler le passage de la barrière cutanée (pH, nature de la membrane du milieu retenant le médicament, etc.), un grand nombre de médicaments ne peuvent pas être administrés au moyen d'un patch passif.

Les recherches ont donc conduit à mettre en application une technique connue depuis près d'un siècle consistant à forcer le passage cutané à l'aide d'un courant électrique qui déplace le médicament sous forme d'ions entre deux électrodes appliquées à peu d'intervalle sur la peau d'un patient. Lorsque le médicament en solution est déjà sous forme d'ions, la technique est désignée par électrophorèse; lorsque le médicament est entraîné par des ions du milieu de dilution, la technique est désignée par iontophorèse. Les dispositifs permettant de mettre en oeuvre ces techniques d'électrophorèse ou d'iontophorèse en traitement ambulatoire sont généralement désignées sous le nom de "patch actif". Le dispositif élémentaire se compose d'un réservoir de médicament au fond duquel est disposée une électrode active, proche mais électriquement isolée d'une deuxième électrode, généralement nommée contre-électrode, les deux électrodes étant reliées entre elles par une source de courant. Contrairement aux modes d'administration traditionnels par voie orale ou parentérale, la durée d'administration iontophorétique ou électrophorétique est longue, ce qui nécessite d'utiliser une source de courant ayant une puissance suffisante, ou plus exactement de définir quel choix doit être privilégié entre l'encombrement de la source de courant et sa durée de vie. Un tel dispositif est par exemple décrit dans le brevet US 4 474 570.

Très rapidement, il est également apparu nécessaire de pouvoir contrôler la diffusion du médicament en fonction du traitement à appliquer en agissant sur la source de courant : le modèle élémentaire de patch actif a alors été complété par un circuit intrégré susceptible d'être programmé en fonction du médicament administré et du traitement devant être appliqué à un patient donné. Un tel patch actif se compose donc des éléments suivants :
- un réservoir de médicament,
- un système d'électrodes,
- une source de courant, et
- un circuit électronique généralement complété par un affichage à cristaux liquides.

Le brevet US 4 640 689 décrit un patch de ce type. Un tel dispositif, destiné à être jeté après chaque usage, s'est rapidement révélé non-satisfaisant à la fois pour des raisons économiques et pour des raisons écologiques. Au fur et à mesure que le circuit électronique s'est perfectionné, il est apparu nécessaire de pouvoir le conserver. Pour éviter de jeter un patch contenant encore du médicament mais dont la source de courant est épuisée, ou inversement ayant encore une réserve de courant mais dont le réservoir de médicament est vide, il est apparu nécessaire de pouvoir séparer la source de courant du réservoir de médicament.

Un dispositif du type précédent est par exemple décrit dans le brevet US 4 708 716. Selon un mode de réalisation, le dispositif décrit est constitué par un bâti réutilisable comportant un circuit électronique, un système d'électrodes, des moyens de fixation au patient, ainsi que deux logements permettant de mettre respectivement en place une pile et un réservoir de médicament. Selon un autre mode de réalisation, la pile est appliquée de l'extérieur, sur le sommet du bâti du patch. Un tel dispositif, dans lequel le système d'électrodes et les moyens de fixation au patient sont conservés, ne permet pas de remplacer facilement un médicament par un autre, ni d'utiliser un même patch pour des patients différents pour des raisons épidémiologiques.

Pour éviter, au moins partiellement, les inconvénients précités, le brevet US 5 135 479 propose un dispositif dans lequel le système d'électrodes et le réservoir de médicament forment une unité séparable du module réutilisable, constitué par le bâti comportant le circuit électronique et un logement pour une pile remplaçable, ledit bâti étant solidaire d'un bracelet de fixation.

Selon un autre dispositif décrit dans le brevet GB 2 239 803, le module jetable, comprenant le système d'électrodes et le réservoir de médicament, contient également une pile fournissant le courant nécessaire pour l'électrophorèse ou l'iontophorèse. Un tel dispositif présente encore les inconvénients évoqués précédemment concernant l'épuisement relatif du réservoir de médicament et de la pile.

Dans tous les cas, les moyens de fixation du patch au patient, notamment par bracelet, étant solidaires du module réutilisable, les risques de contamination demeurent, surtout en milieu hospitalier, lorsqu'un même patch est utilisé par plusieurs patients.

La présente invention a pour objet de remédier aux inconvénients des patchs actifs connus de l'art antérieur, en fournissant un dispositif de traitement ambulatoire pour l'administration transdermique de médicaments par électrophorèse ou iontophorèse composé de trois modules séparables :
- un "module actif" pourvu au moins d'un système d'électrodes et d'un réservoir de médicament,
- un "module-énergie" pourvu d'une source de courant nécessaire pour l'électrophorèse ou l'iontophorèse,
- un module électronique pourvu d'un circuit électronique, d'organes de commande et d'un affichage, dans lequel le module-énergie est situé entre les deux autres modules et comporte, outre une source de courant, d'une part des moyens d'assemblage mécanique et de connexion ou d'interconnexion électriques avec les deux autres modules, et d'autre part des moyens de fixation au corps d'un patient.

Le patch selon l'invention se compose donc d'un module électronique réutilisable, et de deux modules en contact avec la peau, qui sont séparément jetables au bout d'un certain temps d'utilisation. Le patch selon l'invention permet donc également d'améliorer la prophylaxie, particulièrement en milieu hospitalier.

Selon l'invention, le module-énergie contient une ou plusieurs piles assurant un courant suffisant pour une thérapeutique électrophorétique ou iontophorétique pouvant durer jusqu'à une semaine, alors que le réservoir de médicament du module actif a une contenance pouvant être limitée à un traitement de vingt-quatre heures, par exemple pour des raisons de sécurité thérapeutique. Le patch selon l'invention permet donc d'éliminer sélectivement le module actif, ou le module-énergie, selon l'ordre dans lequel leur fonction est épuisée. La nécessité de remplacer le module actif ou le module-énergie peut être signalée sur l'affichage du module électronique.

Selon une autre caractéristique de l'invention, les piles usagées, quelle que soit leur nature, peuvent être facilement extraites du module-énergie et récupérées pour éviter une pollution de l'environnement.

La forme du patch selon l'invention sera généralement fonction du type de piles utilisées et de leur agencement dans le module-énergie, c'est-à-dire en fait de la forme du module-énergie lui-même.

De façon générale, le module-énergie du patch selon l'invention est constitué de deux parties, une platine et un capot, réalisées en un matériau plastique rigide par exemple par moulage ou par thermoformage. Une des faces de la platine est structurée pour maintenir en place le module actif; l'autre face supporte les piles et les organes de connexion ou d'interconnexion entre les piles et les différents modules. Le capot recouvre totalement ou partiellement la face de la platine supportant les piles et les organes de connexion. Ledit capot se trouve également pourvu d'organes permettant de maintenir en place le module électronique. Il est bien évident que, sans sortir du cadre de la présente invention, d'autres constructions peuvent être envisagées. Il est par exemple possible de maintenir le module électronique dans le patch au moyen d'organes disposés sur la platine elle-même. De même, on peut envisager de réaliser le module-énergie par assemblage de plus de deux parties.

Les parties constitutives du module-énergie, incluant les moyens de fixation, sont assemblées selon des techniques usuelles telles que le collage, le soudage, ou l'encliquetage.

Dans le cas où le patch a la forme d'un boîtier circulaire, et où on utilise deux piles bâton (par exemple de type R₁), celles-ci pourront, par exemple, être disposées à plat en V sur la face interne de la platine du module énergie, le secteur circulaire laissé libre pouvant être utilisé pour recevoir le module électronique maintenu en place dans le module-énergie par enfichage, encliquetage ou tout autre moyen approprié. Dans le cas où on choisit d'utiliser deux piles bouton (par exemple de type DA630H), le patch selon l'invention aura par exemple la forme d'un boîtier ovale, les piles étant disposées à plat sur la face interne de la platine du module-énergie, et le module électronique étant ajustable sur le sommet du capot.

Un autre objet de l'invention est de fournir un patch qui possède une sécurité d'utilisation accrue du fait que la séparation du module électronique du reste du patch peut être effectuée rapidement par une manoeuvre simple, qui permet d'inactiver totalement le patch dans le cas où une interruption rapide du traitement est nécessaire.

L'invention sera mieux comprise en référence aux exemples de réalisation illustrés par les dessins annexés dans lesquels :
- la figure 1 représente une vue de dessus d'un patch selon l'invention;
- la figure 2 représente une coupe selon la ligne 2-2 de la figure 1;
- la figure 3 représente une vue en perspective du patch de la figure 1 avec séparation des trois modules;
- la figure 4 représente une vue de dessous de la platine du module-énergie;
- la figure 5 représente une vue de dessus de la platine du module-énergie;
- la figure 6 représente une vue de dessous du capot du module-énergie; et
- la figure 7 représente une vue en perspective, avec séparation des trois modules, d'un autre mode de réalisation d'un patch selon l'invention.

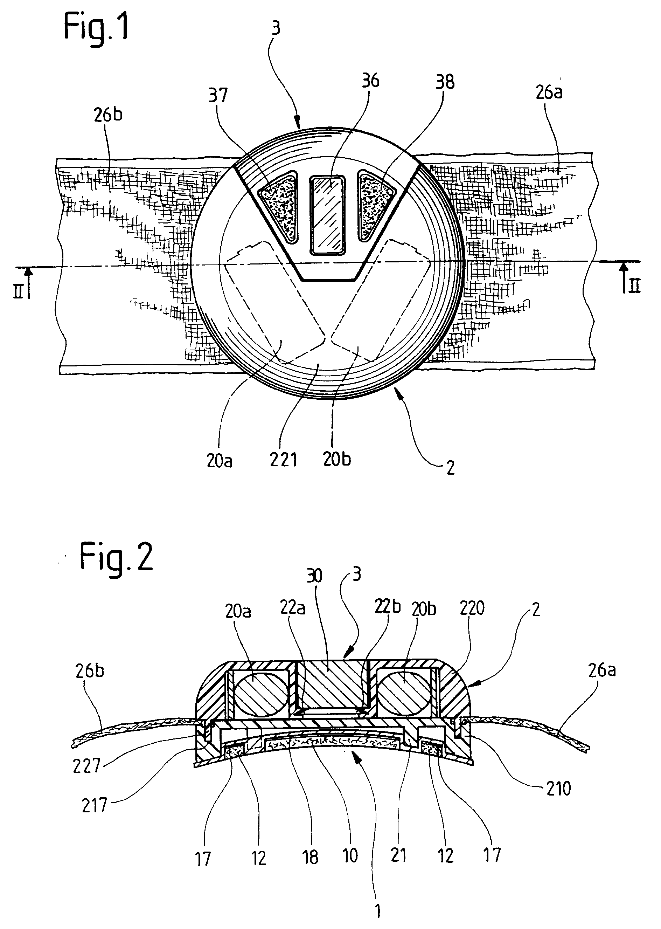

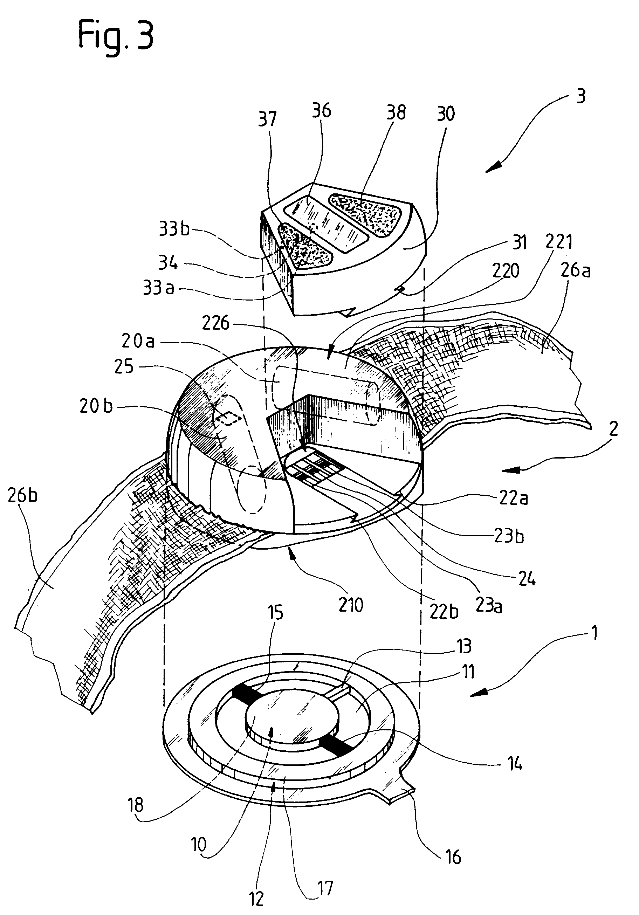

Comme représenté aux figures 1 à 3, et plus particulièrement à la figure 3, un patch selon l'invention est constitué par l'assemblage de trois modules séparables, comprenant un module actif 1 en contact avec la peau du patient, un module-énergie 2 intermédiaire, et un module électronique 3 qui constitue le module réutilisable.

Le module actif 1, de forme générale circulaire, est réalisé en un matériau plastique souple et comporte, sur sa face en contact avec la peau du patient, un système d'électrodes composé d'une électrode active 18 en forme de disque, située au centre et logée au fond d'un réservoir 10 destiné à recevoir le médicament, et d'une contre-électrode 17 en forme d'anneau, logée au fond d'un réservoir annulaire 12 destinée à recevoir un gel conducteur, les réservoirs 10 et 12 étant séparés par un anneau isolant rejoignant les bords extérieurs en regard desdits réservoirs. Sur la face opposée, c'est-à-dire celle qui s'applique sur le reste du patch, les réservoirs 10 et 12 délimitent, de façon complémentaire, une gorge 11 destinée, d'une part à maintenir mécaniquement en place le module actif 1 sur le reste du patch par engagement dans des organes complémentaires de celui-ci, d'autre part à recevoir des contacts 14, 15 reliés respectivement aux électrodes 17, 18 pour assurer la connexion électrique de ces dernières avec le reste du patch. Dans la gorge 11 se trouve également une petite nervure de positionnement 13 radiale, n'autorisant la mise en place du module 1 que d'une façon univoque sur le reste du patch. Ce module 1 est également pourvu d'une petite languette de préhension 16 destinée à faciliter sa mise en place et surtout son extraction lorsqu'il doit être remplacé. Ce module 1 constitue en effet le premier élément jetable lorsque la quantité de médicament du réservoir 10 est épuisée. Ce module 1 peut évidemment être réalisé en donnant aux électrodes toute autre forme connue de l'art antérieur. En tant que tel, le module 1 ne fait pas partie de l'invention; il n'en fait partie que dans la mesure où il coopère avec les deux autres modules.

Le module-énergie 2, réalisé en un matériau plastique rigide, est conformé pour recevoir une source de courant 20 nécessaire pour une administration de médicaments par électrophorèse ou iontophorèse et comporte en outre, d'une part des moyens d'assemblage mécanique 21, 22a, 22b et des moyens de connexion électrique 23a, 23b, 24, 25 avec le module actif 1 et le module électronique 3, d'autre part des moyens de fixation 26a, 26b au corps d'un patient.

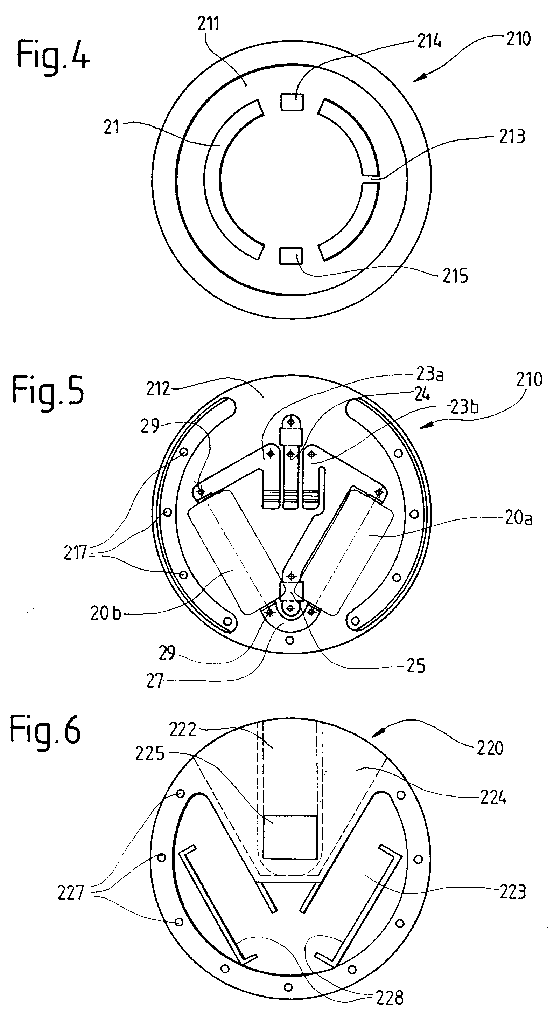

En se référant maintenant également aux figures 4, 5 et 6, on observe que l'assemblage et les connexions avec le module actif 1 sont effectués par des moyens situés sur la partie inférieure du module 2, et l'assemblage et les connexions avec le module électronique 3 sont effectués avec des moyens situés sur sa partie supérieure. A cet effet, le module 2 est constitué de deux parties, obtenues par exemple par moulage ou thermoformage d'un matériau plastique, pour former une platine 210 dont la face externe 211 est orientée vers le module actif 1 et un capot 220 dont la partie externe 221 est destinée à supporter le module électronique 3. La platine 210 et le capot 220 sont assemblés par leurs faces en regard 212, 222, en maintenant en place les moyens de fixation 26a, 26b au corps d'un patient. Dans le patch tel que représenté aux figures 1 à 6, les moyens de fixation 26a, 26b sont constitués par deux bandes de tissu élastique pouvant être réunies à leurs extrémités par une attache de type velcro® pour maintenir le patch autour d'un membre d'un patient. Les bandes 26a, 26b sont rendues solidaires du module 2 par assemblage du capot 220 pourvu à sa périphérie de petits ergots 227 venant s'engager dans des trous correspondants 217 de la platine 210. L'assemblage de la platine 210 et du capot 220 peut également être exécuté par tout autre moyen approprié ne permettant pas le démontage, tel que le collage ou le soudage des deux parties.

En référence aux figures 4 et 5, représentant respectivement les faces externe 211 et interne 212 de la platine 210, on observe que la face externe 211 comporte un anneau en relief discontinu 21 qui vient coopérer avec la gorge 11 du module actif 1 pour maintenir ce dernier mécaniquement en place. Deux discontinuités de l'anneau 21 sont occupées par des contacts 214, 215 situés en regard des contacts 14, 15 du module actif 1.

Une troisième discontinuité 213 de l'anneau 210 est destinée à venir coopérer avec la nervure de positionnement 13 du module 1.

La face interne 212 de la platine 210 est structurée pour recevoir ou maintenir en place les connexions et interconnexions 23a, 23b, 24, 25 entre la source d'énergie 20 et les deux autres modules. Selon le mode de réalisation représenté, la source d'énergie 20 est constituée par deux piles bâton 20a, 20b (par exemple du type R₁), disposées à plat en V, et reliées en série par une connexion 27. Les connexions 23a, 23b permettent de relier les deux pôles de la source d'énergie 20 au module électronique. La connexion 24 est conformée pour relier, par un trou traversant de la platine, une borne de la source d'énergie à une électrode du module actif 1 par l'intermédiaire du contact 215. L'interconnexion 24 est conformée pour relier, par un trou traversant de la platine 210, la deuxième électrode du module actif 1 par l'intermédiaire du contact 214, et une borne de sortie 34 du module électronique 2. Les connexions et interconnexions électriques sont réalisées de façon connue, par exemple par découpe d'une plaque métallique et positionnement sur la platine par fusion de l'extrémité de petits ergots de positionnement 29.

En se référant maintenant aux figures 3 et 6, on observe que le deuxième élément constitutif du module-énergie 2 est constitué par un capot 220 dont une partie de la face interne 222 est ouverte pour former un logement 223 destiné à recevoir la source d'énergie 20, l'autre partie étant fermée par une plaque 224 dans laquelle est ménagée une ouverture 225 donnant accès aux extrémités des connexions 23a, 23b, 24 formant contact avec le module électronique 3. Selon le mode de réalisation représenté, dans lequel la source d'énergie 20 est constituée par deux piles bâton disposées à plat sur la platine, le logement 223 à la forme générale d'un V évidé, des nervures supplémentaires 228 pouvant être prévues pour maintenir les piles en place.

Sur la partie externe 221 du capot 220, les parois externes du logement 223 et la face externe de la plaque 224 délimitent un logement 226 ayant la forme générale d'un secteur circulaire, destiné à recevoir un module électronique 3. Dans le mode de réalisation représenté, le guidage du module électronique 3 dans le logement 226 et son maintien en place sont assurés par des rails 22a, 22b en queue d'aronde venant coopérer avec la partie mâle correspondante 31 située sur la face interne du module électronique 3. Ce guidage et cette fixation du module électronique 3 sur le module-énergie 2 peuvent évidemment, sans sortir du cadre de l'invention, être effectués par tout autre moyen approprié permettant de mettre et d'ôter le module électronique 3 facilement et rapidement. Il est par exemple possible de disposer des rails et des nervures sur les faces en regard du module électronique 3 et du capot 220.

Dans le mode de réalisation représenté, le module électronique 3 a la forme d'un petit boîtier dont le contour est complémentaire de celui du logement 226 du module-énergie 3. Il comporte à l'intérieur un circuit électronique 30, connu en soi pour gérer tous les paramètres usuels d'un traitement par électrophorèse ou iontophorèse, en fonction du médicament à administrer et du patient à traiter. Ces différents paramètres peuvent être initialisés dans le circuit 30 au moyen de boutons de commande 37, 38 et visualisés sur un écran d'affichage 36, ces éléments étant situés sur la face externe du boîtier. Sur sa face interne, le boîtier comporte des contacts 33a, 33b, 34, en regard des contacts situés sur les connexions 23a, 23b et 24 du module-énergie 2. Le module électronique peut posséder sa propre source d'énergie, par exemple une petite pile bouton de longue durée. Il peut aussi posséder un circuit 30 pourvu d'une mémoire non volatile et n'avoir comme source d'énergie que celle du module-énergie 2 lorsque le module électronique est en place. En tant que tel, le module 2 ne fait pas partie de l'invention; il n'en fait partie que dans la mesure où il coopère avec les deux autres modules.

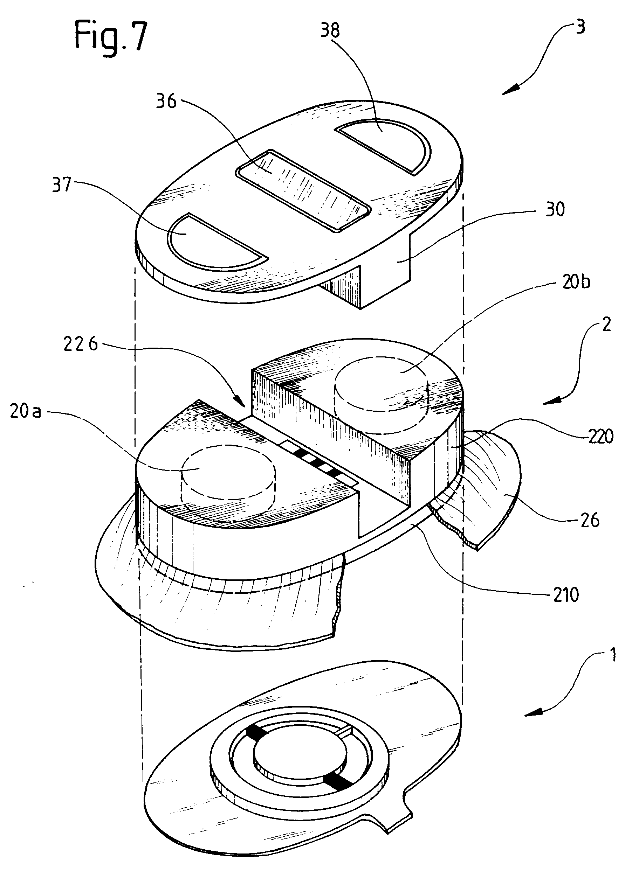

La figure 7 représente un autre mode de réalisation d'un patch selon l'invention, dans lequel le remplacement des deux piles bâton par deux piles bouton (par exemple de type DA630H) conduit à modifier la forme géométrique dudit patch, tout en mettant en oeuvre les mêmes principes de solidarisation mécanique et électrique de trois modules dont seul le module électronique 3 est réutilisable. Selon ce mode de réalisation, le patch a une forme générale ovale. Le module actif 1 épouse cette forme en supportant, sur tout ou partie de sa surface externe, au moins un système d'électrodes et un réservoir de médicaments. Le module-énergie 2, toujours constitué d'une platine et d'un capot, supporte une pile bouton à chacune de ses extrémités, le logement 226 réservé au module électronique 3 ayant alors une position centrale, l'écran d'affichage 36 étant situé au centre et les boutons de commande 37, 38 de part et d'autre dudit écran 36, sur des prolongements du boîtier du module 3 au-dessus du capot 220 du module 2. La fixation au corps du patient est assurée au moyen d'un anneau adhésif 26 maintenu en place entre la platine 210 et le capot 220.

La présente invention, qui vient d'être illustrée par deux exemples pour une meilleure compréhension, n'est évidemment pas limitée à ces modes de réalisation. Sans sortir du cadre de la présente invention, l'homme de l'art est en mesure d'effectuer les changements appropriés dans les formes, les modes de solidarisation mécaniques et électriques ou autre, dans la mesure où le module énergie constitue en définitive la pièce maîtresse du patch selon l'invention.

## Revendications

1. Dispositif de traitement ambulatoire, formé de trois modules séparables, pour l'administration transdermique de médicaments par électrophorèse ou iontophorèse, comportant un premier module actif (1) pourvu au moins d'un système d'électrodes (17, 18) et d'un réservoir de médicaments (10), un deuxième module-énergie (2) pourvu d'une source de courant (20), et un troisième module électronique (3) pourvu d'un circuit électronique (30), d'organes de commande (37, 38) et d'un écran d'affichage (36), caractérisé en ce que le module-énergie (2) est situé entre les deux autres modules (1, 3) et en ce qu'il comporte, outre la source de courant (20) constituée par une ou plusieurs piles, d'une part des moyens d'assemblage mécanique (21, 22a, 22b) et des moyens de connexion ou d'interconnexion électriques (23a, 23b, 24, 25) avec les deux autres modules (1, 2) et d'autre part des moyens de fixation (26a, 26b) au corps d'un patient.

2. Dispositif selon la revendication 1, caractérisé en ce que le module-énergie (2) est constitué par l'assemblage d'une platine (210) dont la face externe (211) est orientée vers le module actif (1), d'un capot (220) dont la partie externe (221) est destinée à supporter le module électronique (3), la platine (210) et le capot (220) étant assemblés par leurs faces en regard (212, 222), en maintenant en place les moyens de fixation (26a, 26b) au corps d'un patient.

3. Dispositif selon la revendication 2, caractérisé en ce que la face externe (211) de la platine (210) comporte un anneau de fixation (21) du module actif (1), pourvu de discontinuités, et deux contacts électriques (214, 215) sur la connexion (25) avec la source d'énergie (20) et sur l'interconnexion (24) avec le module électronique (3), lesdits contacts étant en regard de contacts correspondants du module actif (1).

4. Dispositif selon la revendication 3, caractérisé en ce qu'une des discontinuités (216) de l'anneau (21) permet de positionner le module actif (1) de façon univoque.

5. Dispositif selon la revendication 2, caractérisé en ce que la face interne (212) de la platine (210) est structurée pour recevoir ou maintenir les connexions et interconnexions (23a, 23b, 24, 25) entre la source d'énergie (20) et, d'une part le module actif (1), et d'autre part le module électronique (3).

6. Dispositif selon la revendication 2, caractérisé en ce qu'une partie de la face interne (222) du capot (220) est ouverte pour former un logement (223) destiné à recevoir la source d'énergie (20), et que l'autre partie est fermée par une plaque (224) dans laquelle est ménagée une ouverture (225) pour les connexions et interconnexions (23a, 23b, 24).

7. Dispositif selon la revendication 6, caractérisé en ce que le logement (223) est conformé pour recevoir une source d'énergie (20) constituée par deux piles bâton en série, disposées à plat en V sur la face interne (211) de la platine (210), la plaque (224) occupant l'intérieur du V.

8. Dispositif selon la revendication 6, caractérisé en ce que le logement (223) est conformé pour recevoir une source d'énergie (20) constituée par deux piles-boutons disposées a plat sur la face interne (211) de la platine (210), la plaque (224) étant positionnée entre lesdites piles-bouton.

9. Dispositif selon les revendications 2 et 6, caractérisé en ce que la partie externe (221) du capot (220) comporte un logement (226) délimité par les parois externes du logement (223) de la source d'énergie et par la face externe de la plaque (224), et destiné à recevoir le module électronique (3).

10. Dispositif selon la revendication 9, caractérisé en ce que le logement (226) est pourvu d'organes de guidage et de fixation (22a, 22b) du module électronique (3).

11. Dispositif selon la revendication 10, caractérisé en ce que les organes de guidage et de fixation (22a, 22b) sont constitués par une découpe en queue d'aronde ménagée dans la plaque (224).

12. Dispositif selon la revendication 10, caractérisé en ce que les organes de guidage et de fixation (22a, 22b) sont constitués par des rails ou des nervures disposés sur les parois du logement (226).

13. Dispositif selon la revendication 2, caractérisé en ce que les moyens de fixation (26a, 26b) sont constitués par des bandes de tissu élastique munies à leurs extrémités d'attaches du type velcro®, lesdites bandes étant maintenues par pincement entre la platine (210) et le capot (220).

14. Dispositif selon la revendication 2, caractérisé en ce que les moyens de fixation (26a, 26b) sont constitués par des bandes adhésives entourant tout ou partie du module-énergie (2), lesdites bandes étant maintenues par pincement entre la platine (210) et le capot (220).

15. Dispositif selon la revendication 2, caractérisé en ce que la platine (210) et le capot (220) sont réalisés par moulage ou thermoformage d'un matériau plastique.

16. Dispositif selon la revendication 2, caractérisé en ce que l'assemblage de la platine (210), du capot (220) et des moyens de fixation (26a, 26b) est effectué par collage, soudage ou encliquetage d'organes complémentaires portés par la platine (210) et le capot (220).

## Claims

1. Device for ambulatory treatment, formed of three separable modules, for the transdermic administration of drugs by electrophoresis or iontophoresis, comprising a first active module (1) provided with at least one system of electrodes (17, 18) and one drug reservoir (10), a second power module (2) provided with a power supply (20) and a third electronic module (3) provided with an electronic circuit (30), control organs (37, 38) and a display screen (36), characterized in that the power module (2) is situated between said two other modules (1, 3) and in that it comprises in addition to the power supply (20) formed by one or more batteries, on the one hand mechanical assembly means (21, 22a, 22b) and electrical connection or interconnection means (23a, 23b, 24, 25) with the two other modules (1, 2) and on the other hand means for attaching (26a, 26b) to the body of a patient.

2. Device according to claim 1, characterized in that said power module (2) is formed by the assembly of a plate (210) whose external face (211) is oriented towards the active module (1), and a cover (220) whose external part (221) is intended to support the electronic module (3), the plate (210) and the cover (220) being joined together by their opposite faces (212, 222), thereby holding in place the means for attaching (26a, 26b) to the body of a patient.

3. Device according to claim 2, characterized in that the external face (211) of the plate (210) comprises a ring (21) for fixing the active module (1) provided with discontinuities, and two electrical contacts (214, 215) on the connection (25) with the power supply (20) and on the interconnection (24) with the electronic module (3), said contacts facing corresponding contacts of the active module (1).

4. Device according to claim 3, characterized in that one of the discontinuities (216) of the ring (21) enables the active module (1) to be positioned in a univocal way.

5. Device according to claim 2, characterized in that the internal face (212) of the plate (210) is structured to receive or maintain the connections and interconnections (23a, 23b, 24, 25) between the power supply (20) and, on the one hand the active module (1), and on the other hand the electronic module (3).

6. Device according to claim 2, characterised in that one part of the internal face (222) of the cover (220) is open to form a casing (223) intended to receive the power supply (20), and that the other part is closed by a sheet (224) which houses an aperture (225) for the connections and interconnections (23a, 23b, 24).

7. Device according to claim 6, characterized in that the casing (223) is designed to receive a power supply (20) formed by two rod-shaped batteries in series, placed flat in a V shape on the internal face (211) of the plate (210), the sheet (224) occupying the interior of the V.

8. Device according to claim 6, characterized in that the casing (223) is designed to receive a power supply (20) formed by two button-shaped batteries placed flat on the internal face (211) of the plate (210), the sheet (224) being positioned between said button-shaped batteries.

9. Device according to claims 2 and 6, characterized in that the external part (221) of the cover (220) comprises a casing (226) delimited by the external walls of the casing (223) of the power supply and by the external face of the sheet (224), and intended to receive the electronic module (3).

10. Device according to claim 9, characterized in that the casing (226) is provided with organs for the guidance and fixing (22a, 22b) of the electronic module (3).

11. Device according to claim 10, characterized in that the guidance and fixing organs (22a, 22b) are formed by a dovetail cut arranged in the sheet (224).

12. Device according to claim 10, characterized in that the guidance and fixing organs (22a, 22b) are formed by rails or ribs placed on the walls of the casing (226).

13. Device according to claim 2, characterized in that the attachment means (26a, 26b) are formed by strips of elastic fabric provided at their ends with velcro ®-type fastenings, said strips being gripped between the plate (210) and the cover (220).

14. Device according to claim 2, characterized in that the attachment means (26a, 26b) are formed by adhesive strips encircling all or part of the power module (2), said strips being gripped between the plate (210) and the cover(220).

15. Device according to claim 2, characterized in that the plate (210) and the cover (220) are formed by moulding or thermoforming a plastic material.

16. Device according to claim 2, characterized in that the assembly of the plate (210), the cover (220) and the attachment means (26a, 26b) is achieved by adhesive bonding, welding or snap-fitting of complementary organs carried by the plate (210) and the cover (220).

## Patentansprüche

1. Vorrichtung zur ambulanten Behandlung, die aus drei trennbaren Modulen gebildet ist, um Medikamente transkutan durch Elektrophorese oder Iontophorese zu verabreichen, mit einem ersten aktiven Modul (1), das Wenigstens mit einem Elektrodensystem (17, 18) und mit einem Medikamentenreservoir (10) versehen ist, einem zweiten Energiemodul (2), das mit einer Stromquelle (20) versehen ist, und einem dritten elektronischen Modul (3), das mit einer elektronischen Schaltung (30), Steuerorganen (37, 38) und einem Anzeigeschirm (36) versehen ist, dadurch gekennzeichnet, daß sich das Energiemodul (2) zwischen den beiden anderen Modulen (1, 3) befindet und daß es außer der Stromquelle (20), die aus einer oder mehreren Batteriezellen gebildet ist, einerseits Mittel (21, 22a, 22b) zur mechanischen Anbringung sowie Mittel (23a, 23b, 24, 25) für die elektrische Verbindung oder Zwischenverbindung mit den beiden anderen Modulen (1, 2) und andererseits Mittel (26a, 26b) für die Befestigung am Körper eines Patienten aufweist.

2. Vorrichtung nach Anspruch 1, dadurch gekennzeichnet, daß das Energiemodul (2) durch die Anbringung einer Platine (210), deren äußere Fläche (211) zum aktiven Modul (1) gerichtet ist, und einer Haube (220), deren äußerer Teil (221) dazu vorgesehen ist, das elektronische Modul (3) zu tragen, gebildet ist, wobei die Platine (210) und die Haube (220) an ihren einander gegenüberliegenden Flächen (212, 222) zusammengefügt werden und dabei die Mittel (26a, 26b) für die Befestigung am Körper eines Patienten nicht bewegt werden.

3. Vorrichtung nach Anspruch 2, dadurch gekennzeichnet, daß die äußere Fläche (211) der Platine (210) einen Ring (21) für die Befestigung des aktiven Moduls (1), der Unterbrechungen aufweist, sowie zwei elektrische Kontakte (214, 215) an der Verbindung (25) mit der Energiequelle (20) und an der Zwischenverbindung (24) mit dem elektronischen Modul (1) enthält, wobei sich die Kontakte gegenüber entsprechenden Kontakten des aktiven Moduls (3) befinden.

4. Vorrichtung nach Anspruch 3, dadurch gekennzeichnet, daß eine der Unterbrechungen (216) des Rings (21) ermöglicht, das aktive Modul (1) eindeutig zu positionieren.

5. Vorrichtung nach Anspruch 2, dadurch gekennzeichnet, daß die Innenfläche (212) der Platine (210) so strukturiert ist, daß sie die Verbindungen und Zwischenverbindungen (23a, 23b, 24, 25) zwischen der Energiequelle (20) und einerseits dem aktiven Modul (1) und andererseits dem elektronischen Modul (3) aufnehmen oder halten kann.

6. Vorrichtung nach Anspruch 2, dadurch gekennzeichnet, daß ein Teil der Innenfläche (222) der Haube (220) offen ist, um einen Aufnahmesitz (223) zu bilden, der dazu vorgesehen ist, die Energiequelle (20) aufzunehmen, und daß der andere Teil durch eine Platte (224) verschlossen ist, in der eine Öffnung (225) für die Verbindungen und Zwischenverbindungen (23a, 23b, 24) ausgespart ist.

7. Vorrichtung nach Anspruch 6, dadurch gekennzeichnet, daß der Aufnahmesitz (223) so beschaffen ist, daß er eine Energiequelle (20) aufnimmt, die aus zwei in Serie geschalteten Stabbatterien gebildet ist, die auf der Innenfläche (211) der Platine (210) liegend in Form eines V angeordnet sind, wobei die Platte (224) den Innenraum des V belegt.

8. Vorrichtung nach Anspruch 6, dadurch gekennzeichnet, daß der Aufnahmesitz (223) so beschaffen ist, daß er eine Energiequelle (20) aufnimmt, die aus zwei Knopfbatterien gebildet ist, die liegend auf der Innenfläche (211) der Platine (210) angeordnet sind, wobei die Platte (224) zwischen den Knopfbatterien positioniert ist.

9. Vorrichtung nach den Ansprüchen 2 und 6, dadurch gekennzeichnet, daß der äußere Teil (221) der Haube (220) einen Aufnahmesitz (226) aufweist, der durch die äußeren Wände des Aufnahmesitzes (223) der Energiequelle und durch die Außenfläche der Platte (224) begrenzt ist und dazu bestimmt ist, das elektronische Modul (3) aufzunehmen.

10. Vorrichtung nach Anspruch 9, dadurch gekennzeichnet, daß der Aufnahmesitz (226) mit Führungs- und Befestigungsorganen (22a, 22b) für das elektronische Modul (3) versehen ist.

11. Vorrichtung nach Anspruch 10, dadurch gekennzeichnet, daß die Führungs- und Befestigungsorgane (22a, 22b) durch einen schwalbenschwanzförmigen Ausschnitt gebildet sind, der in der Platte (224) ausgespart ist.

12. Vorrichtung nach Anspruch 10, dadurch gekennzeichnet, daß die Führungs- und Befestigungsorgane (22a, 22b) durch Schienen oder Rippen gebildet sind, die auf den Wänden des Aufnahmesitzes (226) angeordnet sind.

13. Vorrichtung nach Anspruch 2, dadurch gekennzeichnet, daß die Befestigungsmittel (26a, 26b) aus Streifen eines elastischen Gewebes gebildet sind, die an ihren Enden mit Befestigungen des Typs Velcro® versehen sind, wobei die Streifen durch Einklemmen zwischen der Platine (210) und der Haube (220) gehalten werden.

14. Vorrichtung nach Anspruch 2, dadurch gekennzeichnet, daß die Befestigungsmittel (26a, 26b) aus Klebstreifen gebildet sind, die das Energiemodul (2) vollständig oder teilweise umgeben, wobei die Streifen durch Einklemmen zwischen der Platine (210) und der Haube (220) festgehalten werden.

15. Vorrichtung nach Anspruch 2, dadurch gekennzeichnet, daß die Platine (210) und die Haube (220) durch Gießformen oder Thermoformen eines Kunststoffs verwirklicht sind.

16. Vorrichtung nach Anspruch 2, dadurch gekennzeichnet, daß die Anbringung der Platine (210), der Haube (220) und der Befestigungsmittel (26a, 26b) durch Kleben, Schweißen oder Einrasten von komplementären Organen, die von der Platine (210) und von der Haube (220) getragen werden, erfolgt.
